# EUROPEAN PATENT APPLICATION

(11) **EP 3 415 154 A1**
(43) Date of publication of application: **19.12.2018**
(21) Application number: 17305743.1
(22) Date of filing: 16.06.2017
(51) Int. Cl.: A61K 33/06, A61K 33/14, A61K 36/02, A61P 11/02

(54) **NASAL COMPOSITION**

(71) Applicant: SOFIBEL S.A.S., 92300 Levallois-Perret (FR)
(72) Inventor: SAAID, Amina, 60870 RIEUX (FR)
(74) Representative: Fairbairn, Angus Chisholm

(57) **Abstract**

The present disclosure describes a composition for nasal administration comprising: magnesium and one or more components derived from a marine or marine-related plant in admixture in a saline base (e.g., including, but not limited to a saline base comprising seawater). Such compositions can, in some embodiments, decrease inflammation by affecting the release of certain pro-inflammatory neuropeptides.

## Description

### FIELD OF THE DISCLOSURE

The present disclosure relates to compositions for nasal or oral administration and methods for making such compositions. The compositions generally comprise a saline base, a magnesium source, and one or more plant extracts. The disclosure further provides methods of treating patients by administration of such compositions.

### BACKGROUND OF THE INVENTION

Insomnia, whether occasional or regular in nature, affects a significant number of people. Insomnia can include difficulty falling asleep, difficulty staying asleep, waking up too early, and/or waking up feeling unrested. Such abnormal sleep patterns can affect quality of life, leading to various problems including excessive sleepiness during waking hours, difficultly thinking clearly and staying focused, and a depressed mood. Significant sleeping problems, particularly over extended periods of time, can lead to significant mental health problems.

Improving sleep quality is thus important and various types of treatment are regularly employed for this purpose. Treatment can include psychotherapy, medication, and/or behavioral therapy and/or modification. For less severe cases of insomnia, calming/relaxation supplements, including natural sleep aids may be used to aid sleep. For example, magnesium (a metallic element) is a nutrient that serves various functions within the body, including improving sleep quality and reducing nocturnal awakenings.

Magnesium is understood to assist in maintenance of the nervous and muscular balance by reducing release of catecholamine induced by stress (e.g., by oral administration). Magnesium can be derived from various sources and supplements can provide magnesium in various forms, e.g., magnesium aspartate, citrate, lactate, chloride, oxide, and/or sulfate. Magnesium can be found naturally in various foods, and one principal natural source of magnesium is sea water. It would be helpful to provide effective magnesium-containing compositions.

### SUMMARY OF THE DISCLOSURE

The present disclosure provides a composition for nasal or oral administration that can be administered to promote calmness and relaxation. In some embodiments, such compositions may provide assistance to individuals with sleeping disorders (e.g., providing prophylactic treatment). The disclosed compositions can generally comprise one or more magnesium sources in a saline base, and may contain additional components (e.g., a plant-derived component). The unique combination of components in plant-derived component-containing compositions and, in particular, the combination of magnesium and plant-derived component in the disclosed compositions can, in some embodiments, exhibit a synergistic effect with respect to the desired beneficial calming/relaxation effects (based on data provided herein with respect to inhibition of Substance P release). The invention includes, without limitation, the following embodiments.

In a first embodiment, the disclosure provides a composition for oral and/or nasal administration, comprising magnesium and one or more components derived from a marine or marine-related plant in admixture in a saline base.

In a second embodiment, the composition of any preceding or subsequent embodiment is modified such that the on wherein the one or more components derived from a marine plant comprises one or more plant extracts.

In a third embodiment, the composition of any preceding or subsequent embodiment is modified such that the one or more components derived from a marine or marine-related plant further comprises one or more liquid carriers for the one or more plant extracts.

In a fourth embodiment, the composition of any preceding or subsequent embodiment is modified such that the one or more components derived from a marine or marine-related plant are from a plant selected from the group consisting of plants of the asparagopsis genus, plants of the chondrus genus, marine-related plants of the helichrysum genus, marine-related plants of the crithmum genus, and combinations thereof.

In a fifth embodiment, the composition of any preceding or subsequent embodiment is modified such that the one or more components derived from a marine or marine-related plant are from a plant selected from the group consisting of Helichrysum italicum, Crithmum maritimum, Asparagopsis armata, Chondrus crispus, and combinations thereof.

In a sixth embodiment, the composition of any preceding or subsequent embodiment is modified such that the one or more components derived from a marine or marine-related plant are present in an amount of at least about 0.01% by weight based on the composition.

In a seventh embodiment, the composition of any preceding or subsequent embodiment is modified such that magnesium comprises marine magnesium.

In an eighth embodiment, the composition of any preceding or subsequent embodiment is modified such that magnesium is present in an amount of about 1350 g/L or greater, in an amount of greater than 1350 g/L, in an amount of about 1400 g/L or greater, or in an amount of about 1450 g/L or greater (e.g., about 1350 g/L to about 2500 g/L, about 1400 g/L to about 2500 g/L, or about 1450 g/L to about 2500 g/L).

In a ninth embodiment, the composition of any preceding or subsequent embodiment is modified such that the saline base is diluted seawater.

In a tenth embodiment, the composition of any preceding or subsequent embodiment is modified such that the saline base is isotonic or hypertonic.

In an eleventh embodiment, the composition of any preceding or subsequent embodiment is modified such that the composition further comprises hyaluronic acid.

In a twelfth embodiment, the composition of any preceding or subsequent embodiment is modified such that the optional hyaluronic acid comprises a mixture of a low molecular weight hyaluronic acid and a medium molecular weight hyaluronic acid.

In a thirteenth embodiment, the composition of any preceding or subsequent embodiment is modified such that the composition comprises marine magnesium at a concentration of about 1300 g/L or greater (or about 1350 g/L or greater or about 1400 g/L or greater); and an extract of a plant selected from the group consisting of Helichrysum italicum, Crithmum maritimum, Asparagopsis armata, Chondrus crispus, and combinations thereof in admixture with diluted seawater, preferably further comprising hyaluronic acid.

In a fourteenth embodiment, the composition of any preceding or subsequent embodiment is modified such that it is provided in a dispenser adapted for nasal administration, containing the composition of any of these embodiments, the dispenser comprising an aerosol dispenser, a pneumatically pressurized device, a multi-dose metered spray pump, an inhaler, a pump sprayer, a nebulizer, an irrigation system, or a syringe.

In a fifteenth embodiment, a method of preparing the composition of any preceding or subsequent embodiment is provided, comprising: combining a saline base; one or more magnesium sources (which may be provided within the saline base, e.g., where the saline base comprises seawater); and one or more components derived from a marine or marine-related plant.

These and other features, aspects, and advantages of the disclosure will be apparent from a reading of the following detailed description together with the accompanying drawings, which are briefly described below. The invention includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosed invention, in any of its various aspects and embodiments, should be viewed as intended to be combinable unless the context clearly dictates otherwise. Other aspects and advantages of the present invention will become apparent from the following.

### BRIEF DESCRIPTION OF THE DRAWING

In order to provide an understanding of embodiments of the invention, reference is made to the appended figure. The figure is exemplary only, and should not be construed as limiting the invention.

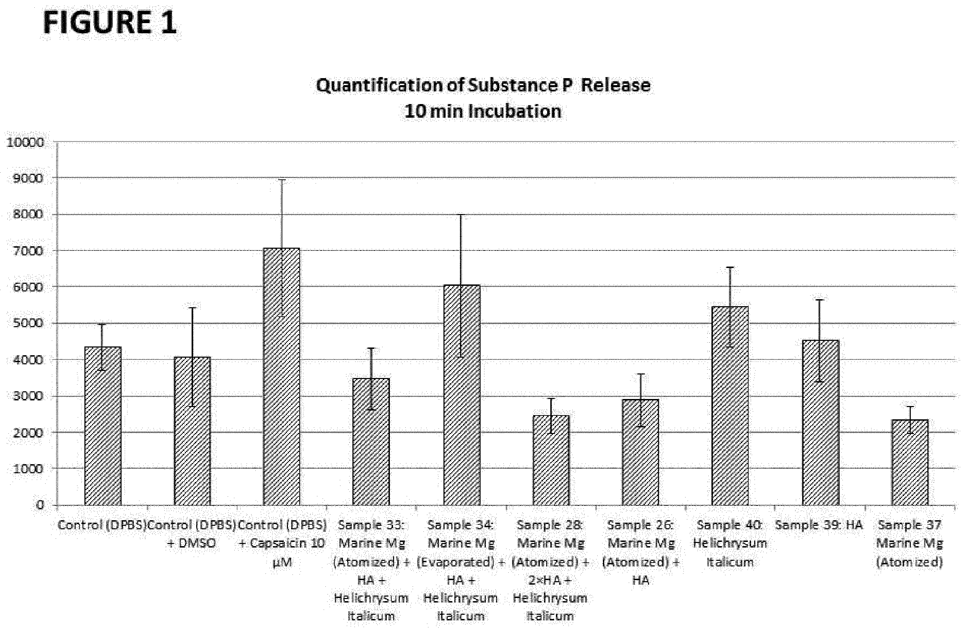

FIG. 1 is a bar graph providing data on Substance P release in the presence of various compositions as disclosed herein with different Mg sources and different HA content.

### DETAILED DESCRIPTION OF THE INVENTION

The invention now will be described more fully herein after through reference to various embodiments. These embodiments are provided so that this disclosure will be thorough and complete, and will fully convey the scope of the invention to those skilled in the art. Indeed, the invention may be embodied in many different forms and should not be construed as limited to the embodiments set forth herein; rather, these embodiments are provided so that this disclosure will satisfy applicable legal requirements. As used in the specification, and in the appended claims, the singular forms "a," "an," and "the" include plural referents unless the context clear dictates otherwise.

The disclosure relates to compositions comprising a saline base, added magnesium, and one or more plant-derived components. The disclosure also relates to devices for administering such compositions, methods of preparing such compositions, and methods of using such compositions (e.g., to treat sleep disorders).

A "saline base" as disclosed herein is generally understood to refer to a solution of sodium chloride (NaCl), and optionally one or more further salts (e.g., calcium chloride and/or CaCl₂) in water. The saline base can be a formulated composition (i.e., formed through addition of salts to substantially pure water) or can be a naturally occurring composition (e.g., seawater) that is used directly or treated in some way, as will be described more fully hereinafter. Saline bases for nasal compositions are generally known in the art and can be employed within the disclosed compositions.

In one or more embodiments, the saline source of the saline base preferably is natural seawater. A saline base according to the present disclosure can thus include any of the materials known to be present in natural seawater. In particular, seawater typically comprises various combinations of dissolved ions, including but not limited to sodium, chlorine, magnesium, vanadium, calcium, potassium, bromide, and/or sulfate ions. A typical composition for naturally occurring seawater is shown below in Table 1. The representative seawater composition is 3.5% by weight (35 g/kg) total salinity. Only elements present in appreciable amounts (i.e., at least 1 ppm) are included, although trace amounts of close to 60 further elements can be present in seawater samples.

**Table 1: Main Elements Present in Typical 35 g/kg Salinity Seawater**

| Element | ppm | | Element | ppm | | Element | Ppm |
|---|---|---|---|---|---|---|---|
| Hydrogen | 110,000 | | Sulfur | 904 | | Nitrogen | 15.5 |
| Oxygen | 883,000 | | Potassium | 392 | | Fluorine | 13.0 |
| Sodium | 10,800 | | Calcium | 411 | | Strontium | 8.1 |
| Chlorine | 19,400 | | Bromine | 68 | | Silicon | 2.9 |
| Magnesium | 1,290 | | Carbon | 28 | | Boron | 4.5 |

The amounts and ratios of these dissolved ions with respect to one another can vary. Seawater (and other saline solutions) can be classified as normal or isotonic (containing about 0.9 w/v NaCl and an osmolality of about 300 mOsm L); hypotonic (containing less than about 0.9 w/v NaCl and less than about 300 mOsm L; or hypertonic (containing greater than about 0.9 w/v NaCl and greater than about 300 mOsmL).

In some embodiments, the saline base in the compositions disclosed herein can comprise a mixture of seawater and water (i.e., diluted natural seawater). Relevant saline bases (e.g., based on seawater) can be isotonic, hypertonic, or hypotonic. Diluted natural seawater can comprise, e.g., about 30 mL to about 80 mL, about 30 mL to about 75 mL, or about 30 mL to about 70 mL of natural seawater in 100 mL total solution. Preferred saline bases are isotonic and contain between about 30 and 35 mL of natural seawater in 100 mL total solution (e.g., about 32 mL of natural seawater in 100 mL total solution) or are hypertonic and contain about 45-80 mL (e.g., 50-75mL) seawater per 100 mL total solution.

The specific source of the seawater can vary. Seawater can, in certain embodiments, be chosen for inclusion within a given composition based on characteristics of the area from which it is obtained. For example, in some embodiments, seawater from regions known to have significant oyster presence is preferred. In some embodiments, seawater from regions with strong ocean currents is preferred. One non-limiting source of natural seawater that is used in certain embodiments disclosed herein is the Baie de Cancale in France.

Seawater can optionally be treated prior to incorporation within the disclosed compositions to modify one or more of the properties thereof. For example, the seawater can, in some embodiments, be advantageously filtered and/or sterilized prior to inclusion (e.g., to remove microorganisms present therein). The seawater can be diluted, concentrated, and/or can be treated so as to modify the ion content/osmolality thereof before being used as or incorporated into a saline base within the disclosed compositions. In some embodiments, seawater is treated to modify the content thereof (e.g., by atomization, concentration, desalination and/or electrodialysis) and is then subsequently diluted by combining the treated seawater with water to provide a saline base for use in the compositions disclosed herein. As such, a saline base for use in the compositions disclosed herein can, in some embodiments, comprise concentrated seawater, atomized seawater, desalinated seawater, and/or electrodialyzed seawater (any of which can be further diluted, e.g., by the addition of water, to form a saline base having the desired dilution).

Electrodialysis (giving electrodialyzed seawater) comprises reducing the content of ions in seawater. In some embodiments, seawater treated by electrodialysis (i.e., electrodialyzed seawater) contains little calcium, but may be rich in other ions, e.g., La, Mg, and Fe ions. In some embodiments, such an electrodialyzed seawater can serve as the "magnesium source" within the compositions disclosed herein, as will be described in further detail herein below, wherein this electrodialyzed seawater can be the sole seawater component of the saline base or can be used in combination with a further seawater (or synthetic analogue thereof) component.

Desalination (giving desalinated seawater) comprises removing at least a portion of the salt (e.g., NaCl) from the seawater. In some such embodiments, seawater treated by desalination (i.e., desalinated seawater) contains decreased Na and Cl, but the overall concentration of other components (e.g., Mg) is typically not significantly changed, although in some desalination processes, the content of other monovalent ions, e.g., potassium, may be decreased or eliminated. In some embodiments, such a desalinated seawater can serve as the "magnesium source" within the compositions disclosed herein, as will be described in further detail herein below, wherein this desalinated seawater can be the sole seawater component of the saline base or can be used in combination with a further seawater (or synthetic analogue thereof) component.

Atomization of seawater (giving atomized seawater) generally comprises atomizing seawater to produce tiny droplets or particles, which can result in the removal of at least a portion of the NaCl present in the sweater. As such, atomized seawater contains decreased NaCl, but generally retains the majority of macro and trace elements originally present in the seawater. In some embodiments, such an atomized seawater can serve as the "magnesium source" within the compositions disclosed herein, as will be described in further detail herein below, wherein this atomized seawater can be the sole seawater component of the saline base or can be used in combination with a further seawater (or synthetic analogue thereof) component.

Concentration of seawater (giving concentrated seawater) generally comprises removing at least a portion of the water from the seawater, e.g., by evaporation. Concentration modifies the overall concentration of dissolved components and changes the osmolality of the seawater and can, in some embodiments, alter the content of certain dissolved species (e.g., ions) with respect to one another. In one particular embodiment, salt water is concentrated (e.g., including, but not limited to, through solar evaporation on a salt marsh), resulting in crystallization and successive deposition of, e.g., calcium salts, sodium, potassium, and magnesium sulfate. The resulting material in this embodiment is a concentrated seawater solution comprising magnesium chloride (with decreased content of, e.g., sodium, calcium, potassium, and sulfate relative to untreated seawater). In some embodiments, such a concentrated seawater can serve as the "magnesium source" within the compositions disclosed herein, as will be described in further detail herein below, wherein this concentrated seawater can be the sole seawater component of the saline base or, more typically, can be used in combination with a further seawater (or synthetic analogue thereof) component.

After any one or more of the treatment methods disclosed herein, the treated seawater can be modified to achieve the desired concentration of liquid/water to make up the saline base for the disclosed compositions. The amount of treated seawater incorporated within the saline base can, in some embodiments, be that amount sufficient to provide the desired amount, e.g., of sodium chloride and/or magnesium. Dilution modifies the overall concentration of dissolved components and changes the osmolality of the seawater-containing solution, but typically does not alter the content of dissolved species (e.g., ions) with respect to one another. Diluting seawater with water (giving diluted seawater) comprises combining seawater with varying amounts of water, e.g., purified, distilled, deionized, demineralized, and/or filtered water. In certain preferred embodiments, seawater is atomized, concentrated, desalinated, or electrodialyzed and then diluted to provide a saline base for use in the disclosed compositions.

In other embodiments, a saline base is synthetically produced by combining water, NaCl, and any one or more components found in natural seawater, as referenced herein above. Commercial saline bases are known and can be used in as-provided or treated form, e.g., by adding additional components thereto, and/or by processing the saline bases as referenced herein above.

The saline base generally makes up a majority of the compositions disclosed herein, e.g., such that at least about 50% by weight, at least about 75% by weight, at least about 80% by weight, at least about 90% by weight, at least about 95% by weight of the composition comprises the saline base. It is understood that in the foregoing examples, the saline base can have a maximum concentration of 99.9% by weight of the composition.

The compositions disclosed herein contain a source of magnesium, which may be present in varying forms and in varying quantities. In some embodiments, where the saline base component comprises seawater, magnesium can be provided by the seawater. In some embodiments, magnesium is intentionally added to the composition (which magnesium can be derived from natural sources or can be synthetically provided). In some embodiments, magnesium is both provided within the saline base (by seawater) and added to the composition, e.g., to obtain a composition with the desired Mg content (as described further herein below).

The form of the magnesium source within the disclosed compositions can be, for example, a magnesium salt (which can, in certain embodiments, be present in the final composition in ionic form, including magnesium ions). Exemplary magnesium salts include, but are not limited to, magnesium chloride, magnesium glucoheptanoate, and a magnesium salt of pyrrolidone carboxylic acid (PCA), e.g., in its L (levorotary) form. Such magnesium salts can be natural or synthetic.

Advantageously, at least a portion of the magnesium present in the composition, and preferably a majority or all of the magnesium present in the composition is natural magnesium (as opposed to synthetic magnesium). Natural magnesium indicates that the magnesium source is naturally occurring, rather than synthetically produced. One exemplary type of natural magnesium that is particularly beneficial for inclusion within the disclosed compositions is "marine magnesium," which is magnesium derived from seawater. Marine magnesium can be directly provided by a seawater saline base in a given composition and/or can be separately provided (by obtaining a magnesium source from a separate seawater sample). In one embodiment, magnesium is provided by concentrating and/or otherwise treating seawater, as referenced above. In one particular embodiment, magnesium is provided by evaporation of seawater on a salt marsh. The concentrated sea water is crystallized and after successive deposition of calcium salts, sodium, and potassium, this method can lead to deposition of most of the magnesium sulfate present in the seawater, which can then be concentrated to give a concentrated solution of natural magnesium chloride. In another embodiment, magnesium is provided by atomizing concentrated sea water, as referenced herein above.

The amount of magnesium can vary in the compositions disclosed herein. Advantageously, compositions are provided that contain magnesium in an amount of about 1300 mg/L or more, about 1350 mg/L or more, greater than 1350 mg/L, about 1400 mg/L or greater, or about 1450 mg/L or greater (e.g., about 1350 g/L to about 2500 g/L, about 1360 mg/L to about 2500 g/L, about 1400 g/L to about 2500 g/L, or about 1450 g/L to about 2500 g/L). Although the application focuses primarily on embodiments wherein the magnesium content is within the above-mentioned ranges, in other embodiments, the amount of magnesium may be below these values, e.g., less than 1300 mg/L (including about 300 mg/L to 1300 mg/L). As such, compositions are provided herein with magnesium values ranging, in some embodiments, from about 300 mg/L to about 2500 g/L. As referenced, this magnesium content may, in some embodiments, represent a combination of magnesium provided by a seawater-containing saline base and one or more magnesium sources added independently to the composition. In one or more embodiments, the compositions can have a magnesium concentration that is greater than the magnesium concentration of natural seawater. As such, the present compositions can comprise seawater and added magnesium, which can be from any source as described above but is preferably marine magnesium. "Added magnesium" indicates the content of magnesium that is included above the amount of magnesium present in natural seawater (or any synthetic saline base that may be used).

The "one or more plant-derived components" present in the disclosed compositions can include various components derived plants and, in particular, components that endow the disclosed compositions with further beneficial effects. Such plant-derived components are commonly in the form of plant extracts, which can be obtained in various manners (e.g., including, but not limited to, liquid extraction, filtration, or centrifugation). Extracts are generally understood to comprise any fraction or isolated or purified molecule from a plant cell. In some embodiments, the extracts are aqueous extracts. In some embodiments, extracts are provided in solid form (e.g., typically concentrated after extraction so as to remove solvent therefrom and include substantially only the extracted components). In other embodiments, extracts contain solvents and ingredients (e.g., carriers) in addition to the plant-derived components (which may be solvents and ingredients used for the extraction, or which can comprise alternative solvents and/or ingredients, e.g., where an extract is obtained, concentrated, and redissolved). For example, plant-derived components may be provided with glycerin as a carrier therefor (e.g., where the extract contains up to about 75% by weight of the glycerin, e.g., about 10 to about 50% by weight glycerin). Typically in such extracts, the plant-derived components themselves make up at least about 25% by weight of the extract.

The plant from which the plant-derived component is derived is advantageously (but not limited to) a marine or marine-related plant. Marine and marine-related plants are found in or near aquatic environments (saltwater or freshwater). In preferred embodiments, such marine and marine-related plants are edible. Marine and marine-related plants include, but are not limited to, algae (e.g., algae of the asparagopsis genus and algae of the chondrus genus), marine plants of the helichrysum genus, and marine plants of the crithmum genus. Certain particularly beneficial plants from which components can be obtained for inclusion within the disclosed compositions are plants that have been demonstrated (qualitatively or quantitatively) to independently affect (e.g., decrease) the release of pro-inflammatory neuropeptides (e.g., Substance P). Such release can demonstrate the ability to provide calming or relaxing effects upon mucosal administration. Advantageously, plant-derived components as used herein do not contain preservatives.

As one specific example, Helichrysum italicum (Immortelle) is a marine-related plant that grows on dry, rocky, or sandy ground around the Mediterranean periphery. An extract of Helichrysum italicum has been shown to stimulate the synthesis of β-endorphins (anti-stress hormones) in fibroblasts. In particular, past studies have demonstrated that application of Helichrysum italicum diluted to 1% by weight to the forearm leads to decreased cortisol in saliva, which was correlated with an increase of β-endorphins. Essential oil derived from Helichrysum italicum is also known to be analgesic.

As another example, Crithmum maritimum (Criste marine) is a marine-related plant also referred to as "Rock samphire" that is edible and that grows in various coastal regions, e.g., on the southern and western coasts of Britany, France and Ireland, and on Mediterranean and western coasts of Europe (including the Canary Islands, North Africa, and near the Black Sea). Crithmum maritimum is known to stimulate the synthesis of β-endorphins (anti-stress hormones) in fibroblasts, with an increase up to 140%. It also inhibits the release of prostaglandin E2 (PGE2), leading to anti-inflammatory and soothing effects.

Asparagopsis armata is a red algae. The cellular juice of this plant can be extracted by successive microgrindings, providing a plant-derived material rich in Mg and silicon. Chondrus crispus is another red algae and can be used as an extract that is rich in arctic peptides such as citrullyl arginine and/or contains various mineral salts, e.g., Na, Ca, Mg, K. and Cl. These exemplary marine plants and extracts thereof are not intended to be limiting; rather, these specific plants are provided as examples of the types of plants and plant extracts generally found to exhibit calming or relaxing effects alone, which effects can be further enhanced as disclosed herein via inclusion within the disclosed compositions.

The amount of plant-derived component incorporated within the disclosed compositions can vary depending on the composition of the plant-derived component. In some embodiments, the plant-derived component is, as noted above, an extract and, in such cases, the extract may contain further ingredients in addition to the plant-derived material, e.g., carriers such as water, glycerin, etc. The amount of such "plant-derived component" (including the plant-derived material and other ingredients, e.g., carriers) can be up to about 10% by weight based on the entire composition, e.g., about 0.001% by weight to about 5% by weight, e.g., about 0.01% by weight to about 5% by weight based on the entire composition. The actual amount of plant-derived material may be, e.g., up to about 1% by weight based on the entire composition, e.g., about 0.001% to about 0.5%, about 0.002% to about 0.1%, about 0.01% to about 0.5%, or about 0.01% to about 0.1% by weight based on the entire composition.

The disclosed compositions can optionally further contain hyaluronic acid. "Hyaluronic acid (HA)" is a polyanionic polysaccharide consisting of N-acetyl-d-glucosamine and beta-glucoronic acid and this term as used herein is understood to comprise not only HA itself, but also salts and derivatives thereof (including, but not limited to, sodium hyaluronate). HA is a common component of nasal compositions, as it is understood to adhere well to nasal mucous membranes and provides nasal hydration. Exemplary HAs that can be included in the compositions disclosed herein include "low molecular weight HA" with a number-average molecular weight Mₙ of no more than about 10⁵ Da, e.g., no more than about 50,000 Da, such as about 20,000 Da to about 50,000 Da. One such low molecular weight HA is prepared by fermentation as disclosed in FR2847818, which is incorporated herein by reference in its entirety. "Medium molecular weight HA," which can be prepared according to biotechnological processes, is understood to have a Mₙ of at least about 10⁵ Da, e.g., between about 100,000 Da and 500,000 Da. In certain preferred embodiments provided according to the present disclosure, the compositions comprise two different HAs, e.g., one low molecular weight HA and one medium molecular weight HA. Where more than one HA is provided in a given composition, the ratio of low to medium MW HA can vary and can be from about 10:1 to about 1:10, e.g., about 5:1 to about 1:5; about 2:1 to about 1:2, or about 1:1.

In some embodiments, the compositions consist essentially only of the aforementioned components, i.e., a saline base (e.g., comprising seawater), a Mg source (which can be provided by the seawater or can be independently added), one or more plant-derived components, and optionally, HA. Certain particular compositions thus consist essentially of seawater, optionally water, optionally a marine Mg source (which can be the seawater itself or seawater-derived Mg such as obtained by atomization or evaporation/concentration), one or more marine plant extracts and, in some embodiments, HA (e.g., one low molecular weight HA and one medium molecular weight HA). One particular such composition contains a saline base in an amount of about 85-99% based on the entire composition (the saline base comprising about 20 to about 40% by weight seawater and about 50 to about 75% by weight water); the plant-derived component (including, e.g., carriers) in an amount of about 1% to about 5% by weight (wherein the plant material itself is present in an amount of between about 0.01% and about 0.5% based on the overall weight of the composition), optionally, hyaluronic acid in an amount of about 0.1 to about 2% by weight (e.g., comprising sodium hyaluronate and hydrolyzed hyaluronic acid), and a magnesium source in an amount sufficient to provide an overall magnesium concentration within the ranges referenced above (e.g., about 1350 g/L or greater, greater than 1350 g/L, about 1360 g/L or greater, about 1400 g/L or greater, or about 1450 g/L or greater). In some embodiments, the present composition may expressly exclude any material other than a saline base (particularly any material not typically found in natural seawater), a magnesium source, a marine plant-derived component, and HA.

However, such compositions are understood to not be strictly limited to containing the foregoing components. Various other components can optionally be included within certain embodiments of the disclosed compositions. For example, types of components generally included in nasal compositions may be included, e.g., including but not limited to, surfactants, pH adjusters (e.g., buffers), cooling or warming agents, flavoring agents, fragrances, vitamins, fruit extracts, herbs, colorants, preservatives, hydrating agents, and the like. In some embodiments, any of the foregoing materials may be expressly excluded from the present composition.

Due to the calming effects of the disclosed compositions, they can, in some embodiments, be generally useful for treatment of mucosal tissue and related conditions that are known to interfere with sleeping in humans. Advantageously, the combination of Mg and one or more plant components in the disclosed compositions is beneficial for the desired inhibition release of Substance P. Substance P is a substance that is released from nerve cells when the nerve cells are stressed. Test data described herein below has demonstrated that the disclosed compositions, comprising Mg, HA, and a saline base are effective at decreasing the release of Substance P, indicating that such compositions decrease such stress.

Although not intended to be limited by theory, it is believed that the compositions disclosed herein may function, at least in part, via activity associated with the transient receptor potential vanilloid 1 (TRPV1) channel, which is a calcium channel involved in many physiological and pathological processes that are inactivated by molecules of the vanilloid class. In vivo, this allows for a hot, painful sensation and can be activated by the capsaicin molecule (responsible, e.g., for the spicy sensation found in chili peppers). In pathological condition, it is one of the pivots of the inflammatory mechanisms involved in the release of neuropeptides. The compositions may exert the desired effect by, e.g., a reduction of exocytosis in general, a reduction of Substance P, a decrease in the half-life of Substance P, an increase in intracellular recycling of Substance P, and/or a decrease in the production of Substance P.

The compositions, as referenced, can thus be provided in the form of pharmaceutical compositions adapted, e.g., for nasal administration. Such pharmaceutical compositions are generally sterile preparations that can be provided using known methods (e.g., by filtration through a suitable membrane filter). Specific forms in which the composition disclosed herein can be provided include, but are not limited to, an aerosol dispenser, a pneumatically pressurized device, a multi-dose metered dose spray pump, an inhaler, a pump sprayer, a nebulizer, an irrigation system (e.g., Neti pot), a syringe, and the like. In some embodiments, the device used is pre-filled with a composition as described herein.

Typical dosing of the disclosed composition can vary. Generally, an effective amount is that amount effective to ameliorate, to some extent, inflamed nasal passages and similar conditions that may inhibit sleeping in humans. The effective amount of the disclosed compositions varies depending on the manner of administration, the age, body weight, general health, and specific sleep problem(s) experienced by the patient.

Where the composition is provided in the form of a nasal spray or nasal drops, a typical dose may be 1-2 applications per nostril. Typically, although not limited thereto, such applications would be done shortly before attempting to sleep (e.g., 1 hour before, 30 minutes before, or 15 minutes before). Compositions and methods for reducing Substance P release, which may result in promoting relaxation and sleep, particularly by providing calming and/or relaxing effects on mucosal tissue, are thus provided. Such methods generally comprise the steps of administering a composition as disclosed herein to a patient (e.g., human) in need thereof.

Embodiments of the present disclosure are further illustrated by the following examples, which are set forth to illustrate the presently disclosed subject matter and are not to be construed as limiting.

### EXPERIMENTAL

### Example 1: Composition screening

The compositions tested according to Example 1 all contained hyaluronic acid in the form of a combination of low and medium molecular weight hyaluronic acid and magnesium from various sources and in different amounts, as provided below in Table 2.

**Table 2: Compositions evaluated in Example 1**

| Reference | Mg source | Origin |
|---|---|---|
| Control (DPBS)^{a} | None | - |
| 9 | Mg from evaporated concentrated seawater^{b} | Natural |
| | Total Mg = 1500 mg/L Mg | |
| 16 | Mg from atomized concentrated seawater^{c} | Natural |
| | Total Mg = 1500 mg/L Mg | |
| 17 | Mg from atomized concentrated seawater^{c} | Natural |
| | Total Mg = 2500 mg/L Mg | |
| 18 | Mg from evaporated concentrated seawater^{b} | Natural |
| | Total Mg = 2500 mg/L Mg | |
| 19 | MgCl₂ | Synthetic |
| | Total Mg = 2500 mg/L Mg | |
| 20 | Mg salt of pyrrolidone carboxylic acid (levorotary form) (L-PCA Mg) | Natural |
| | Total Mg = 2500 mg/L Mg | |
| 21 | Mg glucoheptanoate | Synthetic |
| | Total Mg = 1500 mg/L Mg | |
| 22 | Mg glucoheptanoate | Synthetic |
| | Total Mg = 2500 mg/L Mg | |
| Comp A^{d} | Comparative, commercially available product - isotonic electrodialyzed seawater | |
| Comp B^{d} | Comparative, commercially available product - hypertonic electrodialyzed seawater | |

| | | |
|---|---|---|
| ^{a} Control evaluated alone and with 0.1 % DMSO (in which the capsaicin is diluted). ^{b} Mg from evaporated concentrated seawater obtained by solar evaporation on a salt marsh - after crystallizations and successive deposition of calcium salts, sodium and potassium, and then most of the magnesium sulfate originally contained therein, concentration provides a concentrated solution of marine magnesium chloride ^{c} Mg from atomized concentrated seawater is partly NaCl-free and brings together all macro and trace elements in seawater ^{d} Commercially available products tested as received (no Mg intentionally added, no hyaluronic acid). | | |

In vitro tests of various compositions on cell inflammation were conducted. The tests were based on a co-culture of human keratinocytes (obtained from 3 independent donors via cell extraction from epidermis) and sensory neurons of rats (obtained from 3 rats at 6 days old via removal of cells from dorsal root ganglia). The co-cultures were placed in a multiwell culture plate in fresh medium containing specific growth factor for the co-culture for a few days, after which the supernatant was discarded. The supernatant was then replaced with a medium containing capsaicin (a known inflammation inducer) and a composition as described herein. The capsaicin induces release of pro-inflammatory neuropeptides Substance P and Calcitonin Gene-Related Peptid (CGRP). The resulting treated co-cultures were incubated for 10 minutes and the cell viability and the amount of Substance P was evaluated after this time. Data presented herein below and in the corresponding Figures represents the means of three replicates (n=3). Various mean values are presented with its corresponding +/- standard error of the mean (SEM). Statistical analyses were performed, using Student's t-test (*P<0.05, **P<0.01, and ***P<0.001). Each condition was compared to an untreated culture.

Test data from a colorimetric assay demonstrated that all compositions referenced above were non-toxic (based on values of 70%-100% that of a control co-culture containing only cells with no capsaicin or Mg-containing composition). Each tested composition was found to have no significant effect and the controls indicated that the vehicle for the capsaicin (DMS) is also non-toxic.

The effect of each composition on Substance P release was evaluated by assaying various co-culture supernatants by enzyme-linked immunosorbent assay, ELISA. A decrease in Substance P indicates that the composition provides beneficial effects, i.e., limiting or inhibiting the pro-inflammatory effect of the capsaicin. Standard errors in the analysis were somewhat elevated because the model is highly sensitive with room condition/variation of environment (room temperature, light, movement, etc.); however, these variations are considered as normal and results are interpretable. A strong decrease of Substance P release was noted with all test involving Mg-containing compositions as compared to the controls with and without capsaicin. The two comparative compositions tested provided only a relatively small decrease in Substance P release. According to the data, the best compositions for inhibiting Substance P release contained Mg from evaporated concentrated seawater or atomized concentrated seawater. The data suggested no dose response between the concentration of these seawaters and the inhibition of Substance P release. In fact, a composition containing less atomized sea water exhibited higher inhibition than the composition containing more atomized sea water.

### Example 2: Focused composition screening

Based on the results of Example 1, further testing was done focusing on certain samples, using Mg from evaporated concentrated seawater (to give an overall Mg concentration of 1500 mg/L Mg) or Mg from atomized concentrated seawater (also to give an overall Mg concentration of 1500 mg/L Mg). Additional compositions comprising these two Mg sources with varying amounts of HA and certain such compositions further comprising a plant-derived component (specifically, a Helichrysum Italicum extract containing water and glycerin) were also evaluated, as presented below in Table 3.

**Table 3: Compositions evaluated in Example 2**

| Ref. # | Diluted seawater | Mg from evaporated concentrated seawater^{a} | Mg from atomized seawater | Plant extract | HA (mixture of low and medium MWs) |
|---|---|---|---|---|---|
| 26 | ✔ | | ✔ | - | ✔ |
| 28 | ✔ | | ✔ | Helichrysum Italicum extract^{b} | ✔ |
| | | | | | (2× concentration of Ref. 26 Composition) |
| 33 | ✔ | | ✔ | Helichrysum Italicum extract^{b} | ✔ |
| 34 | ✔ | ✔ | | Helichrysum Italicum extract^{b} | ✔ |
| 37 | ✔ | | ✔ | | |
| 39^{c} | ✔ | | | Helichrysum Italicum extract^{b} | ✔ |
| 40^{c} | ✔ | | | Helichrysum Italicum extract^{b} | |

| | | | | | |
|---|---|---|---|---|---|
| ^{a} Mg from evaporated concentrated seawater obtained by solar evaporation on a salt marsh - after crystallizations and successive deposition of calcium salts, sodium and potassium, and then most of the magnesium sulfate originally contained therein, concentration provides a concentrated solution of marine magnesium chloride ^{b} Helichrysum Italicum extract containing water and glycerin c These samples had a lower concentration of Mg, as they contained Mg only from seawater, with no additional marine Mg added thereto. | | | | | |

Cell viability evaluation was conducted with each composition, and all tested compositions were no more toxic than the samples tested in Example 1. Release of Substance P was then tested for these samples, as in Example 1, certain results of which are shown in FIG. 1. Compositions containing other plant-derived extracts (other than Helichrysum Italicum) were also evaluated, e.g., containing Crithmum Maritum (Compositions 29 and 30), Chondrus Crispus, in the form of an extract containing water, phenoxyethanol, and sorbic acid (Composition 31) and Asparagopsis Armata, in the form of an extract containing methylpropanediol (Composition 32). The algae extracts in these compositions are preserved extracts, and due to the desire to avoid the use of preservatives in the final composition, further studies on compositions comprising Mg from atomized concentrated seawater were conducted using Helichrysum Italicum. All tested plant-derived extract-containing compositions also led to significant decrease in Substance P release (>80% effective) even in conditions of control (without capsaicin). From this data, it is believed that a role independent of the capsaicin is involved (e.g., the TRPV1 channel).

### Example 3: Human nasal epithelial screening

The effects of various compositions formulated as nasal sprays on the mucolytic activity, toxicity profile, and pro-inflammatory reaction was evaluated using a fully differentiated human nasal epithelium cultured at the air-liquid interface. In addition to testing various inventive compositions as disclosed herein, two negative controls were tested (one comprising an untreated culture and the other comprising a saline solution) and three positive controls were tested (one comprising cytomix stimulation for IL-8 release, one comprising Triton X 100 for cytotoxicity evaluation, and one comprising isoproterenol (CBF)). The compositions were maintained in a CO₂ incubator for one week (37°C, 5% CO₂, 100% humidity). Epithelia were reconstituted with cells isolated from 14 nasal donors. To mimic airborne delivery, the compounds were applied to such cultures apically. The cell culture medium was changed every day during 4 days and then frozen for later analysis.

Toxicity effects were evaluated based on: tissue integrity monitoring at Day 0, Day 1, and Day 4 (using quantitative trans-epithelial electrical resistance (TEER) measurements); quantitative determination of cilia beating frequency at Day 1, Day 2, Day 3, and Day 4; qualitative morphology analysis (by examining cellular and tissue integrity under a contrast microscope) at Day 0, Day 1, Day 2, Day 3, and Day 4, and quantitative cytotoxicity monitoring (using a lactate dehydrogenase (LDH) test) at Day 1, Day 2, Day 3, and Day 4. No cytotoxicity was observed for the tested compositions at any testing condition. The TEER data showed some fluctuation; however, none of the tested compositions was determined to affect tissue integrity.

Inflammatory effects were evaluated using quantification of Interleukin 8 (IL-8) as an inflammatory mediator at Day 0, Day 1, Day 2, Day 3, and Day 4. Mucolytic activity was evaluated based on: mucociliary clearance analysis at Day 1 and Day 4 (quantification) and mucin secretion by mucin quantification at Day 1 and Day 4. The effect on apical pH was evaluated by measuring pH at Day 4. Globally, no pro-inflammatory effect was observed for the tested compositions based on IL-8 release analysis. At Day 4, all compositions tested induced an increase of mucociliary clearance. All tested compositions showed a slight increase of mucin release at Day 1 as compared to the saline solution, and most showed a slight increase of mucin release at Day 4.

## Claims

1. A composition for nasal administration, comprising magnesium and one or more components derived from a marine or marine-related plant in admixture in a saline base.

2. The composition of claim 1, wherein the one or more components derived from a marine plant comprise one or more plant extracts.

3. The composition of claim 2, wherein the one or more components derived from a marine plant further comprise one or more liquid carriers for the one or more plant extracts.

4. The composition of any preceding claim, wherein the one or more components derived from a marine plant are from a plant selected from the group consisting of plants of the asparagopsis genus, plants of the chondrus genus, marine plants of the helichrysum genus, marine plants of the crithmum genus, and combinations thereof.

5. The composition of any of claims 1 to 3, wherein the one or more components derived from a marine or marine-related plant are from a plant selected from the group consisting of Helichrysum italicum, Crithmum maritimum, Asparagopsis armata, Chondrus crispus, and combinations thereof.

6. The composition of any preceding claim, wherein the one or more components derived from a marine or marine-related plant are present in an amount of at least about 0.01 % by weight based on the composition.

7. The composition of any preceding claim, wherein the magnesium is marine magnesium.

8. The composition of any preceding claim, wherein the magnesium is present at a concentration of about 1400 g/L or greater.

9. The composition of any preceding claim, wherein the saline base is diluted seawater.

10. The composition of any preceding claim, wherein the saline base is isotonic or hypertonic.

11. The composition of any preceding claim, further comprising hyaluronic acid.

12. The composition of claim 11, wherein the hyaluronic acid comprises a mixture of a low molecular weight hyaluronic acid and a medium molecular weight hyaluronic acid.

13. The composition of claim 1, 2 or 3, comprising:
marine magnesium at a concentration of about 1400 g/L or greater; and
an extract of a plant selected from the group consisting of Helichrysum italicum, Crithmum maritimum, Asparagopsis armata, Chondrus crispus, and combinations thereof;
in admixture with diluted seawater.

14. The composition of claim 13, further comprising hyaluronic acid.

15. A dispenser adapted for nasal administration and containing the composition of any of claims 1-14, the dispenser comprising an aerosol dispenser, a pneumatically pressurized device, a multi-dose metered spray pump, an inhaler, a pump sprayer, a nebulizer, an irrigation system, or a syringe.
